# EUROPÄISCHE PATENTANMELDUNG

(11) **EP 0 803 254 A1**
(43) Veröffentlichungstag der Anmeldung: **29.10.1997**
(21) Anmeldenummer: 97106900.0
(22) Anmeldetag: 25.04.1997
(51) Int. Cl.: A61K 31/60, A61K 47/10, A61K 47/14

(54) **Acetylsalicylsäure enthaltende alkoholische Lösungen zur perkutanen Anwendung, deren Verwendung zur antithrombotischen Therapie sowie Arzneimittel**

(30) Priorität: 25.04.1996 DE 19616539
(71) Anmelder: LUITPOLD PHARMA GmbH, 81379 München (DE)
(72) Erfinder: Traue, Jürgen, Dr., 80992 München (DE); Teubner, Andreas, Dr., 85276 Pfaffenhofen (DE); Wadenstorfer, Elmar, Dr., 80799 München (DE)
(74) Vertreter: Zumstein, Fritz, Dr.

(57) **Zusammenfassung**

Acetylsalicylsäure enthaltende alkoholische Lösungen zur perkutanen Anwendung, deren Verwendung zur antithrombotischen Therapie sowie Arzneimittel

## Beschreibung

Ziel der vorliegenden Erfindung ist eine neue pharmazeutische Zubereitung von Acetylsalicylsäure in Form einer alkoholischen Lösung und deren Verwendung zur perkutanen antithrombotischen Therapie.
Acetylsalicylsäure (nachstehend "ASS" genannt) wird bisher im Stand der Technik hauptsächlich oral appliziert. Dabei stehen in der medizinischen Praxis die therapeutisch genutzten Eigenschaffen eines nicht-steroidalen Antiphlogistikums (NSAID) wie die antiphlogistische, analgetische und antipyretische Wirkung im Vordergrund.
Daneben weist ASS weitere Effekte, wie z.B. eine Hemmung der Thrombozytenaggregation, auf und wird deshalb besonders zur Reinfarktprophylaxe von Hirn- und Herzinfarkten in der Langzeittherapie angewendet. Die Anwendung erfolgt bisher ebenfalls auf peroralem Weg.
ASS (nicht aber Salicylsäure (nachstehend "SS")) führt zu einer irreversiblen Acetylierung und damit dauerhaften Inaktivierung der Cyclooxygenase.
Diese irreversible Hemmung bedeutet für die kernlosen Blutplättchen, die im Gegensatz zu anderen Geweben die Cyclooxygenase nicht durch eine Neusynthese ersetzen können, zugleich eine Hemmung der proaggregatorischen Thromboxansynthese für die gesamte Lebenszeit der Thrombozyten (1 Monat).
Deshalb ist die antithrombotische Wirksamkeit nur für die ASS und nicht für das Hydrolyseprodukt SS gegeben.
Die erforderliche Dosierung zur Verhütung thromboembolischer Komplikationen ist noch nicht endgültig geklärt: Zur Zeit werden je nach Indikation Tagesdosen von 30-500 mg angewendet, wobei die "low dose" Therapie weniger Nebenwirkungen zeigte (Fuster, V. und Mitarb., Circulation **87**, 659-675, 1993).

ASS wird in den Gastrointestinal-Flüssigkeiten, bei der Resorption im Magen und Darm und im Blutplasma rasch zum Hauptmetaboliten SS hydrolysiert.
Die absolute Bioverfügbarkeit von ASS nach peroraler Gabe beträgt aufgrund der präsystemischen Metabolisierung nur ca. 50-70 % (Blume, H. und E. Mutschler, Bioäquivalenz - Qualitätsbewertung wirkstoffgleicher Fertigarzneimittel, Govi Verlag, 2. Ergänzungslieferung 1991, Acetylsalicylsäure).
Die seit einiger Zeit vorgeschlagene transdermale Applikation von ASS ist nun für die antithrombotische Therapie von besonderem Vorteil, da hierbei ASS unter Umgehung des Gastrointestinal-Trakts systemisch appliziert wird.
Bei der antithrombotischen Therapie mit ASS wird bisher fast ausschließlich die orale Verabreichung praktiziert.
Für die lokale Therapie von Erkrankungen der Haut bzw. die Behandlung von Schmerzzuständen, Entzündungen und/oder rheumatischen Erkrankungen mit ASS werden wirkstoffhaltige Salben, Cremes oder Gele eingesetzt.

Hierfür sind folgende Vorschlage bekannt geworden:
- Die FR-A 7 502 651 beschreibt Lösungen von ASS in Ethanol zur transkutanen Behandlung von Schmerzen. Diese ethanolischen Lösungen werden aber in Cremes oder Salben inkorporiert. Resorptionsdaten sind nicht angegeben.
- In US 4 219 548 werden alkoholische Lösungen von ASS beschrieben, die Glycerolmonooleat sowie ein Glykol enthalten. ASS ist in Konzentrationen von 0.5 % bis 10 % enthalten. Die Lösungen sind zur topischen Behandlung von entzündetem Gewebe geeignet. Hauptsächlich werden Hautentzündungen (Akne) als behandelbare Krankheitsbilder beschrieben.
- US 4 460 368 beschreibt ein transdermales System (nachstehend "TDS") zur Applikation von ASS, wobei ASS in wäßriger Lösung zusammen mit Lösungsvermittlern enthalten ist. Angaben zur Stabilität von ASS in der Formulierung und über Plasmaspiegel werden nicht gemacht. Indikation des ASS-TDS sind Schmerz und Entzündung.
- Die EP-A-0 581 587 beschreibt eine Grundlage zur transdermalen Applikation verschiedener pharmazeutischer Wirkstoffe, darunter auch Aspirin (= Acetylsalicylsäure) als antiinflammatorischen Wirkstoff. Die Grundlage besteht obligatorisch aus den drei Komponenten Fettsäureester, Alkohol und Wasser.
- In DE-OS 3 413 052 und US 4 665 063 sind topische Formulierungen zur Behandlung von dermatologischen entzündlichen Erkrankungen beschrieben, darunter auch alkoholische Lösungen von ASS in Konzentrationen > 7 %. Mehrere Lösungen wurden an Patienten mit Hauterkrankungen getestet, wobei keine Angaben zur Resorption von ASS vorliegen.

Für die transdermale Anwendung von Acetylsalicylsäure zur Thrombosetherapie sind folgende Vorschläge bekannt geworden:
- Gemäß WO 92/20 343 werden zur transdermalen Thrombosetherapie alkoholische ASS-Lösungen eingesetzt, die als essentiellen Bestandteil Propylenglykol enthalten. Nach der topischen Applikation erfolgte eine langsame und geringe transkutane Wirkstoffresorption.
- Die DE-OS 4 241 128 bzw. WO 94/13302 beschreiben Pflaster-Systeme für ASS zur Thrombose-Behandlung. Es wird die Anwendung von TDS zur Langzeitanwendung beschrieben, ohne daß Angaben über die Resorption des Wirkstoffs bzw. die Verträglichkeit solcher ASS-Pflastersysteme auf der Haut enthalten sind.

Aufgrund der vorliegenden Erfindung wurde nun überraschenderweise festgestellt, daß ASS aus alkoholischen Lösungen, die ein geeignetes Sekundärlösungsmittel enthalten, schnell und gut resorbiert wird.
Gegenstand der Erfindung sind daher alkoholische Lösungen zur perkutanen Anwendung von ASS, bestehend aus Acetylsalicylsäure bzw. deren Salzen als Wirkstoff, einem einwertigen aliphatischen C₂-C₄-Alkohol als Primärlösungsmittel und einem Ester eines einwertigen aliphatischen C₂-C₆-Alkohols mit einer aliphatischen C₁₂-C₁₆-Monocarbonsäure oder mit einer aliphatischen C₄-C₈-Dicarbonsäure als Sekundärlösungsmittel sowie gegebenenfalls einem cyclischen Terpen als Penetrationsenhancer und/oder Acetanhydrid als Hydrolyse-Inhibitor.
Die Lösung kann sowohl direkt als auch mittels eines geeigneten Trägers appliziert werden.

Das Primärlösungsmittel mit guten Lösungseigenschaften für die Formulierungsbestandteile soll leicht flüchtig sein, während das Sekundärlösungsmittel nicht flüchtig sein darf, um den Wirkstoff in gelöster Form auf und in der Haut zu halten.

Als Primärlösungsmittel für die erfindungsgemäßen alkoholischen Lösungen eignen sich einwertige aliphatische C₂-C₄-Alkohole (insbesondere gesättigte C₂-C₄-Alkohole), wie zum Beispiel Ethanol, Propanol, Isopropanol, Butanol-(1) und Butanol-(2), wobei Propanol und Isopropanol bevorzugt sind. Ganz besonders bevorzugt ist Isopropanol. Der Anteil des Primärlösungsmittels in der Formulierung kann variieren und liegt im allgemeinen bei 10 % bis 90 %, wobei 25 % bis 65 % bevorzugt sind.
Hier und im Folgenden bedeuten (sofern nicht anders angegeben) Prozentangaben Gewichtsprozent und sind bezogen auf die fertige Lösung.

Als Sekundärlösungsmittel im erfindungsgemäßen Sinn eignen sich Ester von aliphatischen gesättigten oder ungesättigten C₁₂-C₁₆-Monocarbonsäuren wie zum Beispiel Laurinsäure, Myristinsäure und Palmitinsäure, wobei Myristinsäure bevorzugt ist, mit einwertigen aliphatischen C₂-C₆-Alkoholen wie zum Beispiel Ethanol, Isopropanol, Isobutanol, Pentylalkohol und Hexylalkohol, wobei Ethanol und Isopropanol bevorzugt sind. Besonders bevorzugt ist Isopropylmyristat.

Gleichfalls als Sekundärlösungsmittel geeignet sind Ester von aliphatischen C₄-C₈-Dicarbonsäuren wie zum Beispiel Bernsteinsäure, Glutarsäure, Adipinsäure oder Pimelinsäure, wobei Adipinsäure bevorzugt ist, mit einwertigen aliphatischen C₂-C₆-Alkoholen wie Isopropanol, Butanol, Pentanol, Hexanol, wobei Butanol bevorzugt ist. Besonders bevorzugt ist Butyladipat.
Der Gewichtsanteil des Sekundärlösungsmittels in der Formulierung kann variieren und liegt im allgemeinen bei 10 % bis 80 %, wobei ein Bereich von 20 % bis 60 % bevorzugt ist.

Als Sekundärlösungsmittel geeignet sind auch Ester aus den genannten Dicarbonsäuren mit einwertigen aliphatischen C₇-C₁₈-Alkoholen, wie Heptanol, Oktanol, Decanol, Laurylalkohol, Myristylalkohol und Palmitylalkohol.

In der erfindungsgemäßen alkoholischen Lösung können gegebenenfalls Penetrationsenhancer vom Typ der cyclischen Terpene, bevorzugt L-Menthol oder Thymol, enthalten sein. Der Gewichtsanteil der Pentrationsenhancer kann zwischen 0.1 % und 5 % liegen, wobei ein Bereich von 0.5 % bis 2 % bevorzugt ist.

Gegebenenfalls kann Acetanhydrid zur Verhinderung der Hydrolyse des Wirkstoffs ASS in den erfindungsgemäßen Lösungen enthalten sein. Bevorzugt wird hierbei der Einsatz von Acetanhydrid in einer Konzentration von 0.001 % bis 0.15 %.

Der Wirkstoff ASS ist üblicherweise in Konzentrationen von 1 % bis 15 %, bevorzugt von 5 % bis 15 % und besonders bevorzugt von 10 % in der alkoholischen Lösung enthalten.
Die alkoholischen ASS-Lösungen können zur Applikation in einen geigneten Träger eingearbeitet oder auch direkt auf die Haut aufgetragen werden.

Gegenüber den aus WO 92/20343 bekannten pharmazeutischen Zubereitungen läßt sich mit den erfindungsggemäßen alkoholischen ASS-Lösungen eine unerwartet bessere Penetration von ASS erzielen, wie Untersuchungen der Anmelderin ergaben.
Ferner ergaben Untersuchungen der Anmelderin, daß bei Verwendung der erfindungsggemäßen alkoholischen Lösungen signifikante ASS-Plasmaspiegel erreicht werden.
Die nachfolgenden Beispiele erläutern die Erfindung näher, ohne sie im Umfang darauf zu beschränken.

### Beispiel 1

1 kg ASS wurde in einer Mischung aus 3.2 kg Isopropanol und 5.8 kg Butyladipat (Cetiol B) gelöst und nach Klarfiltration in 200 Schraubflaschen mit Sprühkopf abgefüllt.

### Beispiel 2

1 kg ASS wurde in einer Mischung aus 4.5 kg Isopropanol und 4.5 kg Butyladipat (Cetiol B) gelöst und nach Klarfiltration in 200 Schraubflaschen mit Sprühkopf abgefüllt.

### Beispiel 3

1 kg ASS und 0.1 kg L-Menthol wurden in einer Mischung aus 3.15 kg Isopropanol und 5.75 kg Butyladipat (Cetiol B) gelöst. Der Lösung wurden 10 g Acetanhydrid zugesetzt. Nach Klarfiltration erfolgte die Abfüllung in 200 Schraubflaschen mit Sprühkopf.

### Beispiel 4

1.2 kg ASS wurde in einer Mischung aus 6.45 kg Isopropanol und 2.35 kg Isopropylmyristat gelöst und nach Klarfiltration in 200 Schraubflaschen mit Sprühkopf abgefüllt.

### Beispiel 5

1.2 kg ASS wurde in einer Mischung aus 4.8 kg Isopropanol und 4.5 kg Isopropylmyristat gelöst und nach Klarfiltration in 200 Schraubflaschen mit Sprühkopf abgefüllt.

### Beispiel 6

1 kg ASS wurde in einer Mischung aus 5 kg Isopropanol und 4 kg Cetiol B gelöst und nach Klarfiltration in 200 Schraubflaschen mit Sprühkopf abgefüllt.

### Beispiel 7

1 kg ASS wurde in einer Mischung aus 4.9 kg Isopropanol und 4 kg Isopropyladipat (Cremogen IP) gelöst. Der Lösung wurde 0.1 kg L-Menthol zugesetzt. Nach einer Klarfiltration erfolgte die Abfüllung in 200 Schraubflaschen mit Sprühkopf.

### Untersuchungen am Mäusehaut-Resorptionsmodell (MHR-Modell), Tabelle 1

Ca. 500.0 mg der erfindungsgemäßen ASS-Lösung wurden jeweils auf excidierte Haut haarloser Mäuse aufgetragen, die in einer Franz-Diffusionszelle aufgespannt war. Im Akzeptormedium Puffer pH 7.2 erfolgte zu den angegebenen Zeiten die Wirkstoff-gehaltsbestimmung mittels HPLC-Analytik, so daß der Wirkstoff-Flux in µg/cm² x h berechnet werden konnte.
Zum Vergleich wurden die im Patent WO 92/20 343 beschriebenen Formulierungen von 10 % ASS in Propylenglykol/Isopropanol (1.7/1), Formulierung A, bzw. Propylenglykol/Ethanol (1.7/1), Formulierung B, getestet (Tab. 1).
In den Untersuchungen wurde übetraschenderweise festgestellt, daß aus der nach Beispiel 1 hergestellten erfindungsgemäßen Zubereitung die Acetylsalicylsäure wesentlich schneller und nachhaltiger durch die Haut resorbiert wird als nach Applikation der 10%igen Lösungen von ASS in den Vergleichsformulierungen nach WO 92/20 343 (Tab. 1).

Durch die Anteile des Primär- bzw. Sekundärlösungsmittels ließ sich der ASS-Flux durch die Mäusehaut steuern. Ein weiterer Zusatz von Menthol zur erfindungsgemäßen alkoholischen Lösung von ASS entsprechend Beispiel 3 führte zu einer nochmaligen starken-Penetrationsverbesserung der ASS.

Auch die erfindungsgemäße alkoholische Lösung gemäß Beispiel 4 mit Isopropylmyristat (nachstehend "IPM" genannt) als Sekundärlösungsmittel ergab einen wesentlich höheren ASS-Flux als die Vergleichsformulierungen.

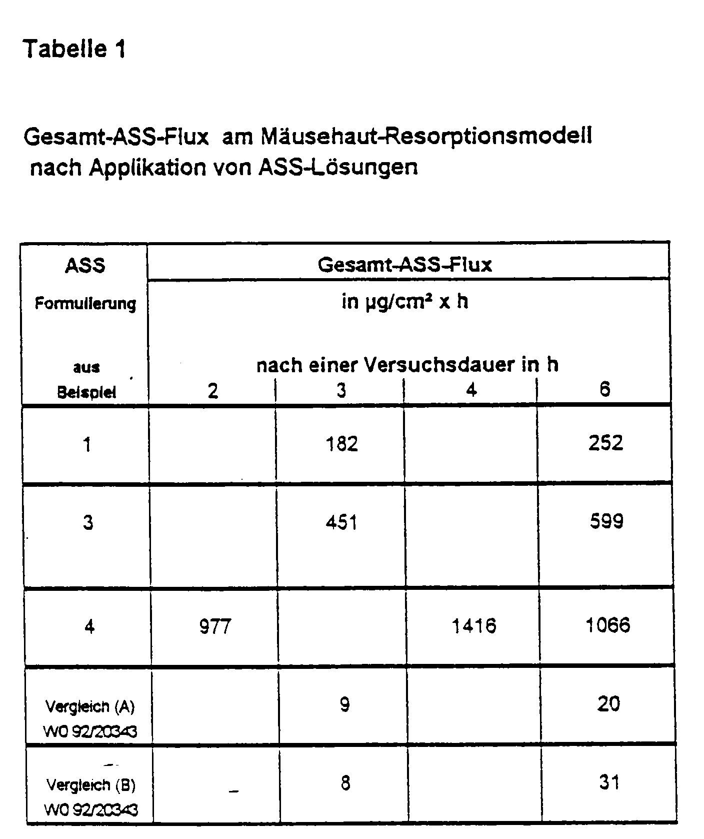

### Testung der ASS-Resorption an Kaninchen, Abbildung 1

### Kutane Applikation der ASS-Lösung aus Beispiel 1 (100 mg ASS/kg)

Eine Stunde nach der Applikation der 10%igen ASS-Lösung aus Beispiel 1 auf den Rücken der 5 Kaninchen wurde bereits bei 2 Tieren und nach zwei Stunden bei allen untersuchten Tieren ASS im Plasma gefunden. Bedingt durch die kutane Wirkstoffresorption kam es innerhalb der ersten 6 Versuchsstunden zu einem langsamen Anstieg der ASS-Plasmaspiegel auf ca. 20 µg/ml.
Die Summe der Konzentrationen von ASS und SS, berechnet als ASS, war ein Maß für die Gesamtmenge resorbierter ASS. Dieser Gesamt-ASS-Plasmaspiegel, lag nach sechs Stunden bei 75 µg/ml.
Vom Metaboliten SS wurden mit geringer Verzögerung ab der 2. Stunde jeweils vergleichbare bzw. höhere Plasmaspiegel als von ASS gemessen - maximal wurde die doppelte SS-Konzentration festgestellt.
Auch 24 Stunden nach der kutanen Applikation wurde noch eine relativ hohe ASS-Konzentration von ca. 20 µg/ml gemessen, was eine länger andauernde ASS-Resorption aus einem Hautdepot belegt.

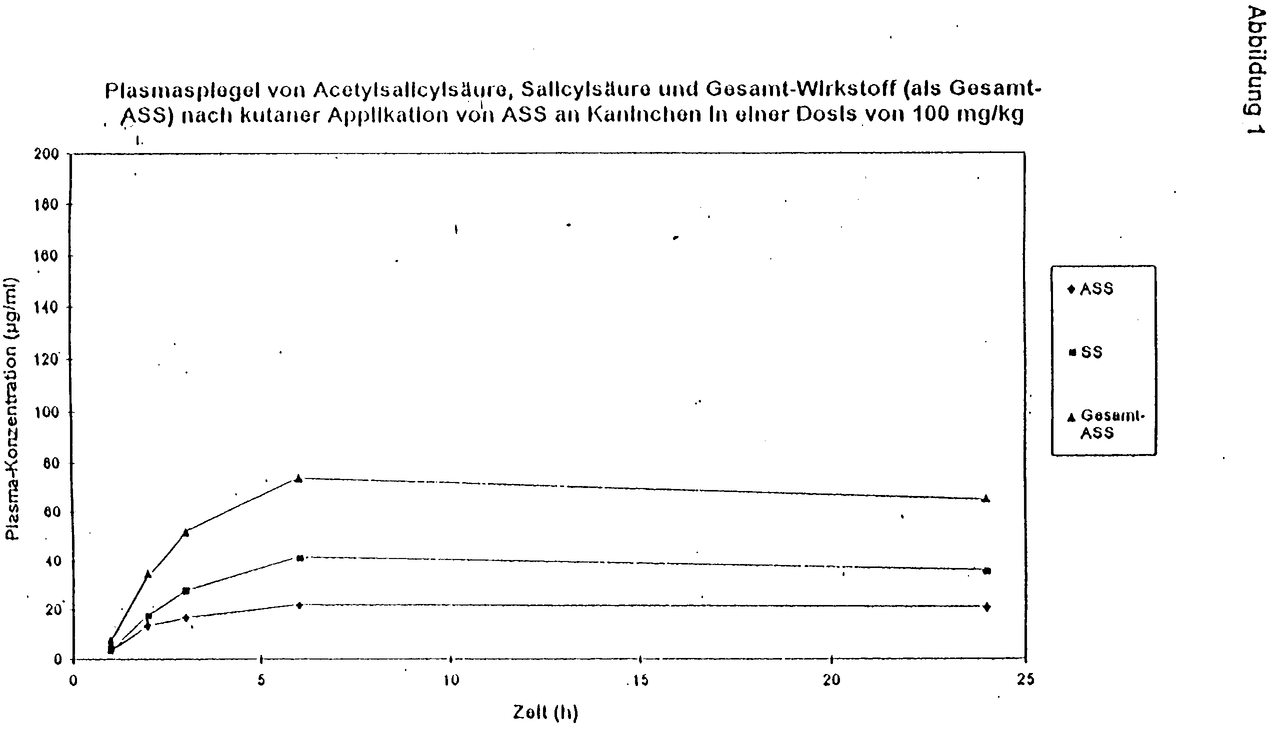

## Patentansprüche

1. Alkoholische Lösungen zur perkutanen Anwendung, bestehend aus Acetylsalicylsäure bzw. deren Salzen als Wirkstoff, einem einwertigen aliphatischen C₂-C₄-Alkohol als Primärlösungsmittel und einem Ester eines einwertigen aliphatischen C₂-C₆-Alkohols mit einer aliphatischen C₁₂-C₁₆-Monocarbonsäure oder mit einer aliphatischen C₄-C₈-Dicarbonsäure als Sekundärlösungsmitttel sowie gegebenenfalls einem cyclischen Terpen als Penetrationsenhancer und/oder Acetanhydrid als Hydrolyse-Inhibitor.

2. Alkoholische Lösungen gemäß Anspruch 1 dadurch gekennzeichnet, daß das Primärlösungsmittel Isopropanol ist.

3. Alkoholische Lösungen gemäß einem der vorstehenden Ansprüche dadurch gekennzeichnet, daß Acetylsalicylsäure bzw. deren Salze in einer Konzentration von 1 % bis 15 % , das Primärlösungsmittel in einer Konzentration von 10 % bis 90 % und das Sekundärlösungsmittel in einer Konzentration von 10 % bis 80 % enthalten ist.

4. Alkoholische Lösungen gemäß einem der vorstehenden Ansprüche dadurch gekennzeichnet, daß der als Sekundärlösungsmittel vorhandene Ester einer aliphatischen C₄-C₈-Dicarbonsäure auch ein Ester mit einem einwertigen aliphatischen C₇-C₁₈-Alkohol sein kann.

5. Alkoholische Lösungen gemäß einem der vorstehenden Ansprüche dadurch genennzeichnet, daß das cyclische Terpen L-Menthol oder Thymol ist.

6. Alkoholische Lösungen gemäß einem der vorstehenden Ansprüche dadurch gekennzeichnet, daß das cyclische Terpen in einer Konzentration von 0.1 % bis 5 % enthalten ist.

7. Alkoholische Lösungen gemäß einem der vorstehenden Ansprüche dadurch gekennzeichnet, daß Acetanhydrid in einer Konzentration von 0.001 % bis 0.15 % enthalten ist.

8. Verwendung der alkoholischen Lösungen gemäß Anspruch 1 bis 7 zur perkutanen antithrombotischen Therapie.

9. Verwendung der alkoholischen Lösungen gemaß Anspruch 8 durch direktes Aufbringen auf die Haut oder durch Einarbeiten der Lösung in einen geeigneten Träger.

10. Arzneimittel zur perkutanen antithrombotischen Therapie bestehend aus oder enthaltend eine alkoholische Lösung gemäß einem der Ansprüche 1 bis 7.
